# EUROPÄISCHE PATENTANMELDUNG

(11) **EP 2 206 483 A1**
(43) Veröffentlichungstag der Anmeldung: **14.07.2010**
(21) Anmeldenummer: 09075015.9
(22) Anmeldetag: 12.01.2009
(51) Int. Cl.: A61F 7/00, A61H 15/00

(54) **Anti-Falten-Stift**

(71) Anmelder: Uterstädt, Andrea, 13589 Berlin (DE)
(72) Erfinder: Uterstädt, Andrea, 14193 Berlin (DE)
(74) Vertreter: Boeckh, Tobias

(57) **Zusammenfassung**

Die Erfindung betrifft ein Handgerät zur kosmetischen Behandlung von Hautpartien, insbesondere im Gesichtsbereich bevorzugt zur Glättung beziehungsweise bevorzugt kosmetischen Behandlung der Augenpartie.

## Beschreibung

Die Erfindung betrifft ein Handgerät zur kosmetischen Behandlung von Hautpartien, insbesondere im Gesichtsbereich bevorzugt zur Glättung beziehungsweise bevorzugt kosmetischen Behandlung einer Faltenpartie.

Im Stand der Technik ist bekannt, dass durch Bakterien oder Viren hervorgerufene Hautirritationen, wie zum Beispiel Akne oder Herpespickel, mithilfe von Vorrichtungen, insbesondere Handgeräten, behandelbar sind, die einen Wärmeeintrag in die Haut initiieren oder unterstützen, indem sie kleinere Hautareale für einen definierten Zeitraum auf 40 bis 60 °C erhitzen.

Diese Temperatur reicht im Regelfalle aus, um das Hautirritationen verursachende Agens - wie zum Beispiel Viren, Bakterien aber auch in Insektengifte (zum Beispiel Mückenstiche) - zu denaturieren oder anderweitig zu zerstören und somit die betroffene Hautpartie kosmetisch zu behandeln und zu glätten. Diese sogenannten "Pickelstifte" des Standes der Technik können aber nur mit mäßigem Erfolg verwendet werden, um Hautpartien die um den Akne- oder Insektenstichbereich angeordnet sind, zu glätten und zu behandeln. D. h. Hautareale, die durch normale Alterungsprozesse oder beispielsweise stärkere Sonneneinstrahlung - also primär nicht durch Akne, Herpes oder Insektenstiche - verändert sind, können mit den bekannten Vorrichtungen nicht kosmetisch behandelt werden.

So ist mit der US 4763657 im Stand der Technik ein Handgerät zur thermisch induzierten Akupunktur beschrieben worden, mit dem Hautbereiche erwärmt werden können. Diese Druckschrift offenbart allerdings nicht, das mit der Vorrichtung Hautpartien insbesondere kosmetisch behandelt werden können.

Die WO 01/34074 beschreibt eine Einrichtung zur lokalen thermischen Behandlung von Insektenstichen und -bissen, ohne jedoch auf eine mögliche Verwendung im kosmetischen Bereich hinzuweisen.

In der DE 19752282 wird ein Gerät zur Diagnose und Therapie durch Wärmezufuhr und Wärmeentzug an Körperflächen mittels eines steuerbaren, von einer Spannungsversorgung gespeisten Peltier-Elements offenbart, mit welchem auf das Bindegewebe Einfluss genommen werden soll.

Alle diese Geräte des Standes der Technik erlauben es dem Anwender aber nicht, besonders empfindliche Hautpartien, wie beispielsweise die im Augenbereich so zu behandeln, dass diese straffer, jünger oder ausgeruhter wirken.

Aufgabe der Erfindung war es daher Vorrichtungen, Verfahren und Verwendungen bereitzustellen, die die Nachteile des Standes der Technik nicht aufweisen.

Es war völlig überraschend, dass die erfindungsgemäße Vorrichtung, beziehungsweise ihre Verwendung gemäß der unabhängigen Ansprüche die Nachteile des Standes der Technik nicht aufweist. Vorteilhaft Ausführungsformen ergeben sich aus den Unteransprüchen.

Ein Aspekt der Erfindung betrifft eine Vorrichtung, bevorzugt in Form eines Handgerätes umfassend ein Heizelement, dass beispielsweise als elektrische Heizplatte und/oder Platine ausgeführt sein kann, die durch eine Spannungsquelle gespeist und nach circa 10 Minuten, bevorzugt 8 Minuten abgeschaltet wird; für diese 8 Minuten wird eine konstante, jedoch variierbare Temperatur, bevorzugt zwischen 39°C und 45°C, gehalten. Weiterhin umfasst diese Vorrichtung einen Bereich mit einer Kaltfunktion, beispielsweise eine Bergkristallkugel mit einem bevorzugten Durchmesser von 22mm, oder einer Vertiefung, in die beispielsweise selbstgemachte Eiswürfel oder konfektionierte Kühlakkus eingebracht werden können. Beim Kühlen der Faltenpartie wird die Temperatur von -5°C bevorzugt nicht unterschritten.

Das Heizelement kann eingesetzt werden, um Kunststoff, Glas oder halbedelsteinhaltige Materialien, bevorzugt in flächiger Pillenform, erwärmt.

Bevorzugt kann das Heizelement in einer Aufheizphase auf eine maximale Temperatur im Bereich von 35 bis 55 Grad Celsius (°C), bevorzugt 40 bis 42°C erhitzt werden und die maximale Temperatur in der Heizphase für einen Zeitraum von 0,5 bis 10 Minuten, vorzugsweise von 3 bis 8 Minuten aufrecht erhalten.

Während dieser Zeit werden durch das Heizelement bevorzugt die Halbedelsteine, die in Pillenform vorliegen und in das eine Ende des Handgerätes eingebracht sind, erhitzt. Das erfindungsgemäße Handgerät umfasst in einer bevorzugten Ausführungsform ein Griffteil welches so dimensioniert ist, dass es mithilfe der Finger einer Hand gut umfasst werden kann. Sofern die Faust den Griffteil umschließt, ragen über und unterhalb der Faust ein oberes und ein unteres Ende des Handgerätes dieser bevorzugten Ausführungsform hinaus.

An den jeweiligen Enden des Gerätes können zum einen die in Pillenform vorliegenden Halbedelsteine (Wärmeseite) angebracht sein und am anderen Ende - der sogenannten Kühlseite - zum Beispiel eine Bergkristallkugel, die vor der Anwendung bzw. in der Zeit der Anwendung der Heißfunktion für mind. 10 Minuten in ein Eisfach gelegt wird. Die Kälte speichert der Stein selbst, sowie in Verbindung damit eine kleine Luftkammer unter der Kugel, die die Kälte bevorzugt für ca. 4 Minuten hält. Statt der Bergkristallkugel können auch selbstgemachten Eiswürfel oder konfektionierten Kühlakkus vorliegen, so dass beide Seiten des Handgerätes durch einfaches Drehen problemlos im Gesichtsbereich zum Einsatz kommen können, indem die erfindungsgemäße Vorrichtung mit Hilfe von Handbewegungen mit den gewünschten Hautpartien in Kontakt gebracht wird.

Die jeweiligen Enden des Gerätes sind so gestaltet, dass sie eine größtmögliche Menge an Kälte- oder Wärmeenergie auf die Haut einbringen können. Bevorzugt sind die Enden des Gerätes im Wesentlichen plan, wobei sie aber auch nach außen oder innen gewölbt sein können, um sich den Konturen des Gesichtes optimal anpassen zu können.

Zur besseren Handhabung des Gerätes kann es in einer bevorzugten Ausführungsform vorgesehen sein, dass die beiden Enden des Gerätes in die gleiche oder aber in unterschiedliche Richtungen abgewinkelt vorliegen.

In einer weiteren bevorzugten Ausführungsform weist das Handgerät ein Teilevolumen im Bereich von 40.000 bis 50.000 mm² und eine Formoberfläche von circa 50.000 bis 70.000 mm² auf.

Es war überraschend, dass diese technischen Parameter ein besonders gut handhabbares Gerät bedingen, welches mit Erfolg eingesetzt werden kann, um den Energiefluss - d. h. Kälte oder Wärme - optimal auf die Haut aufzubringen. Bei dem Energiefluss kann es sich im Sinne der Erfindung um das Wärmehandling oder Kältehandling im Bereich von Hautpartien, bevorzugt des Gesichtes handeln.

Zum optimalen Wärmehandling können die Edelsteine, beziehungsweise Halbedelsteine, an einem im Wesentlichen planen oder leicht gewölbten Ende des Gerätes insbesondere in Pillenform so eingebracht werden, dass verschiedene Edelsteine oder Habedelsteine eine mehr oder weniger einheitliche Fläche ergeben, die durch das Heizelement erwärmt wird.

Selbstverständlich ist es auch möglich, dass ein planes Ende der Vorrichtung vorliegt, in welches eben abschließend mehrere pillenförmige Halbedelsteine platziert vorliegen. Bevorzugt können weiterhin Edelsteine in Form von kleinen Kugeln verwendet werden. Durch mindestens ein Heizelement können diese alle einheitlich oder verschieden erwärmt werden.

In einer weiteren bevorzugten Ausführungsform besteht die erfindungsgemäße Vorrichtung aus mindestens einer Gehäuseunterschale und einer Gehäuseoberschale, die bevorzugt auch unlösbar geschnappt oder verschweißt werden können. Durch das Zusammenfügen der beiden Schalen entsteht ein Raum in dem beispielsweise die Batterie, die Batteriekontakte sowie die Platine mit der Ladebuchse und das Heizelement eingebracht werden können. Über einen Silikonschalter, beispielsweise in der unteren Gehäuseschale kann die Platine und ein an ihr befestigter Ein- und Ausschalter mit der äußeren Gehäuseschale so verbunden werden, dass das Heizelement über den Silikonschalter ein- und ausgeschaltet werden kann.

Das Heizelement ragt bevorzugt in das Ende der erfindungsgemäßen Vorrichtung, in welchem beispielsweise eine zu erwärmende Endkappe aus Aluminium oder einem anderen Material positioniert ist, in die die zu erwärmenden Halbedelsteine eingebracht sind. Das andere und somit gegenüberliegende Ende der Vorrichtung kann eine Kappe, bevorzugt aus einem Leichtmetall, bevorzugt Aluminium mit einem Gewinde bilden, durch welches ein Kühlakku oder ein Eiswürfel beispielsweise im Filmdosenformat in die Vorrichtung eingebracht und fixiert werden kann. Der Energiefluss erfolgt an diesem Ende der Vorrichtung bevorzugt über einen Bergkristall, der die Kälte nach außen und somit bei der Anwendung auf die Haut leitet.

In einer weiteren bevorzugten Ausführungsform umfasst das Gehäuse Elemente der Elektronik, wobei das Gehäuse bevorzugt zwei Schalen, zwei Aluklappen, bevorzugt drei bis sechs Mineralien für das Heizen und Wärmen bzw. ein bis drei Mineralien für das Kühlen umfasst, genauso wie einen Silikonschalter und einen Netzsteckerverschluss. Die Elektronik in dieser bevorzugten Ausführungsform umfasst eine Platine, ein Heizelement, den Schalter, die Ladebuchse für Netzstecker, Batteriekontakte sowie bevorzugt ein bis drei Leuchtdioden zur Schalteranzeige.

Die Erfindung betrifft demgemäß auch einen Kit, welcher das erfindungsgemäße Handgerät, bevorzugt ein Netzteil und/oder zwei bis vier Batterieakkus in einer Verpackung umfasst, die beispielsweise eine Kunststoff-Schatulle mit Deckel sein kann, die im Inneren ein Tiefziehteil, Schaumeinlagen, Stoff oder ähnliches aufweist, um die bevorzugte Variante des Handgerätes sowie ein Netzteil bzw. die Batterieakkus und kosmetische Lotionen und Wässer aufzunehmen.

Somit betrifft die Erfindung auch die Verwendung der erfindungsgemäßen Vorrichtung, bevorzugt in Form eines Handgerätes, zur Behandlung von Falten und Hautpartien, zur Straffung bzw. Festigung des Bindegewebes durch Heiß-Kalt Wechselbehandlung, Bakterienabtötung durch die Wärme, das Öffnen der Poren durch die Hitze. Somit wird das Einschleusen von Seren ermöglicht und die anschließende Kaltfunktion schließt die Poren und somit wird das Serum in der Haut gehalten, insbesondere im Gesicht, bevorzugt der Augenpartie bis hin zum Augenknochen.

Augenpartien im Sinne der Erfindung sind alle Hautareale, die um das Auge herum angeordnet sind bis hin zum "Augenknochen" , wie beispielsweise der Bereich der sogenannten Tränensäcke oder andere. Zur Anwendung der Vorrichtung in diesen Regionen wird das Gerät bzw. die erfindungsgemäße Vorrichtung speziell vorbereitet. Diese Vorbereitung kann beispielsweise in einer bevorzugten Ausführungsform der Erfindung darin bestehen, dass der oder die Kühlakkus in ein Eisfach oder einen Kühlschrank gelegt werden. Wahlweise können auch Eiswürfel aus einer mitgelieferten Form, die Bestandteil des erfindungsgemäßen Kits sein kann, hergestellt werden. Die Batterieakkus können während der Vorbereitungsphase der erfindungsgemäßen Verwendung mittels Netzteils aufgeladen werden. Derartige einzelne Vorbereitungsschritte der Verwendung haben allerdings einen fakultativen Charakter.

In einer weiteren bevorzugten Ausführungsform der Erfindung wird vor Anwendung des bevorzugten Handgerätes eine Emulsion zubereitet, die Collagen, Hyaluron und verschiedene Aminosäuren umfasst. Die Emulsion wird auf bestimmte Regionen des Gesichtes aufgetragen.

Für die Nutzung der erfindungsgemäßen Vorrichtung wird ggf. ein Netzstecker eingesteckt und die gewünschte Wärme durch Schalterstellung des Stromschalters bereitgestellt. Bei Behandlungen der Faltenpartien werden in einer bevorzugten Ausführungsform maximal 42 Grad Celsius für maximal acht Minuten bereitgestellt. Vorteilhafterweise weist die erfindungsgemäße Vorrichtung drei Stufen zur Regulierung der Wärme auf.

Der erfindungsgemäße Heiß-Kalt-Stift bzw. die erfindungsgemäße Vorrichtung werden verwendet, um beispielsweise die Augenpartien zu erwärmen, um anschließend das genannte Serum aufzutragen. Selbstverständlich ist es auch möglich, zunächst das Serum aufzutragen und die entsprechende Hautregion, beispielsweise die Faltenpartie, anschließend mittels der Vorrichtung zu erwärmen. In diesem Falle wird das Handgerät gemäß der Erfindung verwendet, um mithilfe des einen Endes der Vorrichtung, an welchem sich die Edelsteine befinden, das zuvor aufgetragene Serum mittels Wärme in die Haut einzubringen. Es war völlig überraschend, dass die Kombination aus Wärme und dem Serum, umfassend eine Zusammenstellung aus Aminosäuren, Collagen und Hyaluronsäure zu einer deutlichen kosmetischen Verbesserung beispielsweise der Faltenpartie führt. Die Erwärmung der Hautpartien erfolgt mithilfe der beheizten Edelsteineinsätze, die beispielsweise Aventurin, Rosenquarz oder Amethyst oder jegliche Art von Mineralien sein können.

Zum Abschluss der Behandlung erfolgt nach thermischer Einbringung des Serums eine Kälteapplikation über das bevorzugt kalte Ende des Handgerätes/Heiß-Kalt-Stiftes. Die Kaltfunktion der erfindungsgemäßen Vorrichtung wird bevorzugt mithilfe einer gekühlten Bergkristallkugel realisiert. Diese kühlenden Materialien wirken direkt auf die Haut.

Analog zu den erwärmten Edelsteinen wird bei Abschluss der Behandlung der gekühlte Bergkristall eingesetzt, um die Haut zu straffen und die Poren, die das Serum aufgenommen haben können, zu schließen. Nach Beendigung der kosmetischen Behandlung können die bevorzugten Kühlakkus entnommen und wieder in das Eisfach gelegt werden. Die Geräteköpfe können unter fließendem Wasser gereinigt werden und die Geräte in die Schatulle oder die Anordnung des Kits abgelegt werden.

Die erfindungsgemäße Vorrichtung kann zur Optimierung ihrer Anwendung verschieden gestaltet sein. Die Anwendungsflächen für die Wärme- und Kältebehandlung sollten weitestgehend plan oder leicht abgerundet sein, so dass ein guter Wärme- oder Kälteeintrag durch Auflegen oder leichtes Einmassieren (Darüberfahren) der entsprechenden Gesichtspartie erzielt werden kann. Die Vorrichtung, insbesondere in Form eines Handgerätes, sollte eine im Wesentlichen schlanke, gut in der Hand liegende Form aufweisen. Die Anwendungsflächen für die Wärme- und/oder Kältebehandlung, die an den jeweiligen Enden der im Wesentlichen in einer zylindrischen Grundform gestalteten erfindungsgemäßen Vorrichtung angebracht sind, können so angeschrägt sein, dass sie beide bei entsprechender Handhabung dem Gesicht zugeneigt sind. Selbstverständlich kann es auch vorgesehen sein, dass die jeweiligen abgeschrägten Enden in unterschiedliche Richtungen weisen.

Im Folgenden soll die Erfindung anhand einiger Abbildungen näher erläutert werden, ohne auf diese beschränkt zu sein.
- Fig. 1: Draufsicht einer Kugelpfanne mit drei Kugeln
- Fig. 2: Schnitt einer Kugelpfanne mit drei Kugeln
- Fig. 3: Draufsicht einer Kugelpfanne mit sechs Kugeln
- Fig. 4: Schnitt einer Kugelpfanne mit sechs Kugeln
- Fig. 5: Draufsicht einer separaten Kugelpfanne mit jeweils einer Kugel
- Fig. 6: Schnitt einer separaten Kugelpfanne mit jeweils einer Kugel
- Fig. 7: Draufsicht einer in Reihe angeordneten separaten Kugelpfanne mit jeweils einer Kugel
- Fig. 8: Schnitt einer in Reihe angeordneten separaten Kugelpfanne mit jeweils einer Kugel
- Fig. 9: Draufsicht von drei Kugeln, die über eine Achse gehalten werden und in Reihe angeordnet sind
- Fig. 10: Schnitt von drei Kugeln, die über eine Achse gehalten werden und in Reihe angeordnet sind
- Fig. 11: Draufsicht von einer Kugelpfanne mit einer gelagerten Kugel
- Fig. 12: Schnitt von einer Kugelpfanne mit einer gelagerten Kugel
- Fig. 13: Draufsicht von einer Kugeln, die über eine Achse gehalten wird
- Fig. 14: Schnitt von einer Kugeln, die über eine Achse gehalten wird
- Fig. 16: Eine Sofort-Wärme-Kompresse
- Fig. 17: Eine Kälteeinleitung am Stiftende bzw. Edelsteinkugeln
- Fig. 19: Sofort-Kälte-Kompresse auf Amoniumnitrat-Basis
- Fig. 21: Peltier-Element
- Fig. 22: 1. Konstruktive Ansicht einer bevorzugten Ausführungsform
- Fig. 23: 2. Konstruktive Ansicht einer bevorzugten Ausführungsform
- Fig. 24: 3. Konstruktive Ansichten
- Fig. 25: Zeigt zwei Seitenansichten einer bevorzugten Ausführungsform der erfindungsgemäßen Vorrichtung in Form eines Handgerätes
- Fig. 26: Zeigt eine schematische Darstellung des technischen Konzeptes der Vorrichtung nach Anspruch 1
- Fig. 27: Zeigt einen bevorzugten Kit
- Fig. 28 - 30: Die Fig. 28 - Fig. 30 zeigen Varianten von bevorzugten Ausführungsformen der erfindungsgemäßen Vorrichtung

Im Folgenden soll die erfindungsgemäße Vorrichtung anhand mehrerer Beispiele und verschiedener Vorzugsvarianten näher erläutert werden, ohne auf diese Beispiele oder Vorzugsvarianten beschränkt zu sein. Sofern bestimmte Merkmale im Folgenden als obligatorisch beschrieben wurden, bezieht sich dies allein auf die Beispiele oder die beschriebene Vorzugsvariante.

### Aufgabenbeschreibung:

Entwicklung eines "Anti- Falten-Stiftes" zum einschleusen eines Anti- Falten-Serums.

Das Gel wird von Hand auf das komplette Gesicht (auch unter der Augenpartie) aufgetragen, danach wird mit dem warmen Stiftende das Serum einmassiert.

Dieses Ende ist mit drei drehbaren Edelsteinkugeln (Aventurin, Rhodonit, Ozeanachat) bestückt, über die die Wärme geleitet wird.

Die Edelsteine müssen beweglich sein, d.h. müssen sich drehen können (das muss jedoch nicht elektronisch geschehen, es reicht Beispielsweise ein Kugellager)

Die Wärme des Stift- Endes bewirkt, dass sich die Poren öffnen, damit das Gel besser aufgenommen werden kann.

Die Temperatur am warmen Ende ist regelbar, darf aber die Temperatur von 45°C nicht überschreiten.

Nach einem maximalen Dauerbetrieb von 8 Minuten am Stück sollte sich die Warmfunktion automatisch abschalten, damit Verletzungen oder Überreizungen vermieden werden.

Zur Beruhigung der Haut und zum Schließen der Poren besitzt der Stift ein kaltes Stiftende, das mit einem drehbaren Edelstein (Bergkristall) bestückt ist.

Die Temperatur am kalten Ende, darf die Temperatur von -5°C nicht unterschreiten.

Nach einem maximalen Dauerbetrieb von 8 Minuten am Stück sollte sich die Kaltfunktion automatisch abschalten.

Die Energieversorgung erfolgt vorzugsweise durch eine Batterie/ Akku oder, falls aufgrund der erforderlichen Kapazität notwendig, per Stecker- Netzteil mit Kleinspannung, dieses müsste jedoch abnehmbar sein (ähnlich wie bei einem Handy).

Der Mittelbereich dieses kosmetischen Stiftes beinhaltet die erforderlich Heiß-/ Kaltfunktion, sowie einen ON-/ OFF- Betrieb mit einer kleinen Signalleuchte visuell den Betrieb ersichtlich macht.

Der Stift muss leicht zu reinigen sein, da das Serum sich beim einmassieren um die Steine verteilt.

Da die Anwendung im Gesicht und auch unterhalb der Augen erfolgt, ist eine ergonomisch günstige, möglichst kleinvolumige Ausführung anzustreben.

Untersuchung über die Möglichkeiten drei Edelsteinkugeln rollend in einem Kopf zu platzieren. (Wärmeseite)

Da die Massage meisten im Gesicht bzw. bei den Augenpartien erfolgt, ist eine kleine Bauweise erwünscht.

Wir haben uns bevorzugt für einen Kugeldurchmesser von Ø 5mm entschieden da 0 von 3mm oder kleiner Schwierigkeiten im Handling bei der Monatage oder bei der Reinigung hervorrufen würden.

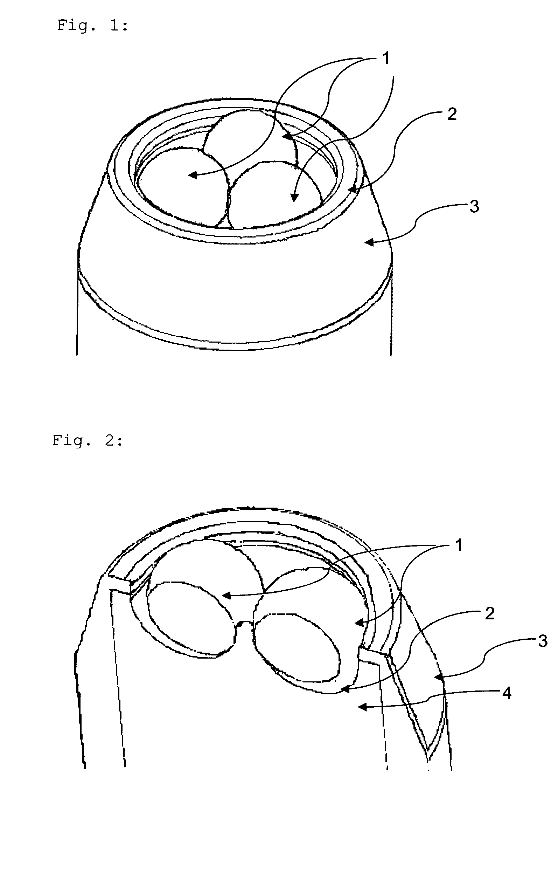

**Fig. 1 und Fig. 2** zeigen eine Vorzugsvariante, bei der drei Kugeln 1 in einer Kugelpfanne 2 angeordnet sind, wobei Fig. 1 eine Draufsicht zeigt und Fig. 2 ein Schnitt durch die Kugelpfanne 2 darstellt.

### Vorzugsvariante 1

Ø 5mm
Anzahl 3
Außen- 0 ca. 14 mm

Die drei Kugeln 1 laufen in einer Kugelpfanne 2. Eine Kappe 3 und eine Kontur in der Mitte verhindern das Herausfallen der Kugeln 1. Die Aufheizung der Kugeln 1 bzw. des Endes des Stiftes kann durch eine in der Kugelpfanne 2 eingeschlossene Heizplatte 4 erfolgen.

Vorteile: Kugeln 1 können sich in alle Richtungen drehen; Kappe 3 kann zur Reinigung abgenommen werden; Das Ende kann unter fliesendes Wasser gehalten werden; Der Gesamtdurchmesser des Kopfes ist gering ca. 13 mm.

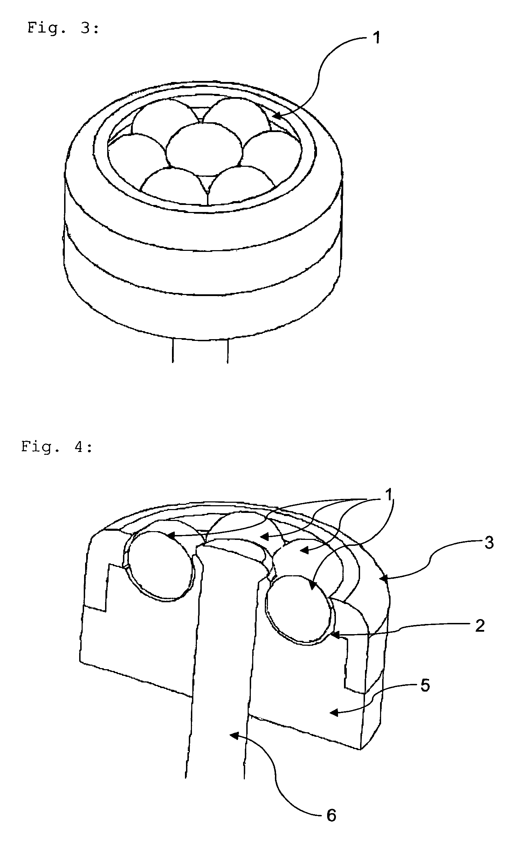

**Fig. 3 und Fig. 4** zeigen eine Vorzugsvariante, bei der sechs Kugeln 1 in einer Kugelpfanne 2 angeordnet sind, wobei Fig. 3 eine Draufsicht und Fig. 4 einen Schnitt der Kugelpfanne 2 zeigen.

### Vorzugsvariante 2

Ø 5mm
Anzahl 6
Außen- 0 ca. 22mm

Die sechs Kugeln 1 laufen in einer Kugelpfanne 2. Eine Kappe 3 und eine ausgeprägte Kontur in der Mitte verhindern das Herausfallen der Kugeln 1. Die Aufheizung des Endes des Stiftes 6 bzw. Wärmeabgabe erfolgt über eine Wärmeplatte 4 in der Mitte der Kugelanordnung.

Vorteile: Kugeln 1 können sich in alle Richtungen drehen; Kappe kann zur Reinigung abgenommen werden; Das Ende kann unter fliesendes Wasser gehalten werden; Reibung der Kugeln 1 untereinander ist geringer da mehr Freiraum für jede einzelne Kugel 1 vorhanden ist; Temperatureinleitung geht über eine separate Fläche (Energiezufuhr geringer).

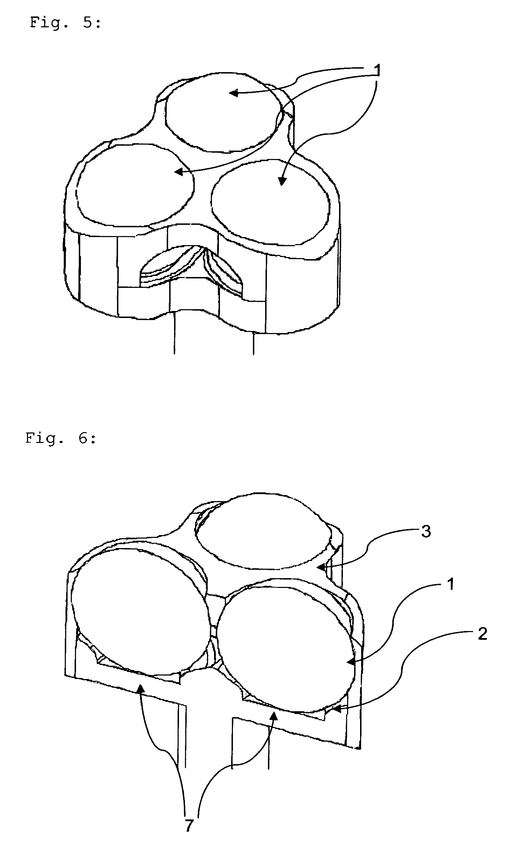

**Fig. 5 und Fig. 6** zeigen eine Vorzugsvariante, bei der drei Kugeln 1 in einer separaten Kugelpfanne 2 angeordnet sind, wobei Fig. 5 eine Draufsicht und Fig. 6 einen Schnitt der Kugelpfanne 2 zeigen.

### Vorzugsvariante 3

Ø Kugel 5mm
Außen-Ø ca. 14mm
Anzahl 3

Die drei Kugeln 1 laufen in einer jeweils separaten Kugelpfanne 2. Eine Kappe 3 mit Löchern verhindert das Herausfallen der Kugeln 1. Die Aufheizung des Endes des Stiftes bzw. Wärmeabgabe erfolgt entweder über jeweils eine Wärmeplatte 7 unter den Kugeln 1 oder bei einem größeren Abstand der Kugel 1 in der Mitte über einen Stempel 6 bzw. könnte auch die Kappe 3 selber als Heizelement fungieren.

Vorteile: Kugeln 1 können sich in alle Richtungen drehen; Kappe 3 kann zur Reinigung abgenommen werden; Das Ende kann unter fliesendes Wasser gehalten werden; Reibung der Kugeln 1 untereinander ist nicht vorhanden, Temperatureinleitung geht über eine separate Fläche (Energiezufuhr geringer) oder durch Aufheizung der Kugeln 1 von unten (Wärmeleitung geringer)

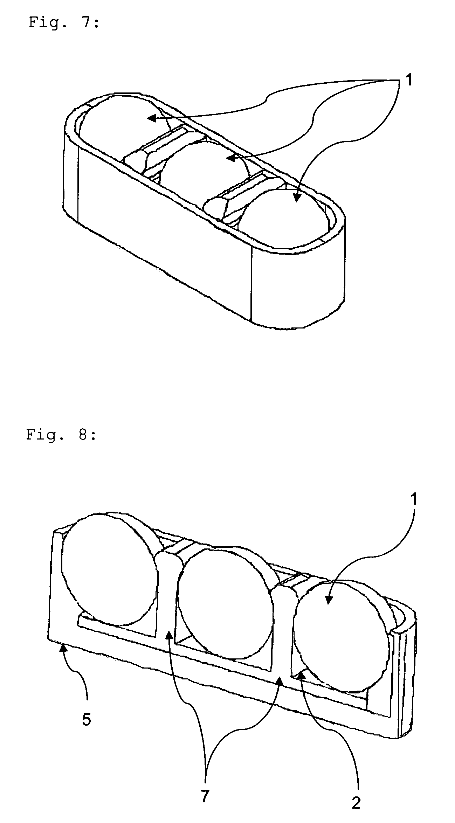

**Fig. 7 und Fig. 8** zeigen eine Vorzugsvariante, bei der drei Kugeln 1 in einer in Reihe angeordneten separaten Kugelpfanne 2 angebracht sind, wobei Fig. 7 eine Draufsicht und Fig. 8 einen Schnitt der Kugelpfanne 2 zeigen.

### Vorzugsvariante 4

Ø 5mm
Anzahl 3

Die drei Kugeln 1 laufen in einer jeweils separaten Kugelpfanne 2, sind aber in Reihe angeordnet. Die symmetrisch aufgebauten Hälften und die Heizelemente 7 verhindern nach der Montage ein Herausfallen der Kugeln 1. Die Aufheizung des Endes des Stiftes bzw. Wärmeabgabe erfolgt über entweder jeweils eine Wärmeplatte 7 unter den Kugeln und/oder über Stempel welche sich zwischen Kugel 1 und 3 befinden.

Vorteile: Kugeln 1 können sich in alle Richtungen drehen; Das Ende kann unter fliesendes Wasser gehalten werden; Reibung der Kugeln 1 untereinander ist nicht vorhanden, Temperatureinleitung geht über eine separate Fläche (Energiezufuhr geringer) oder durch Aufheizung der Kugeln 1 von unten (Wärmeleitung geringer); Durch den Reihenaufbau kann eine min. Breite von ca. 7 mm erreicht werden.

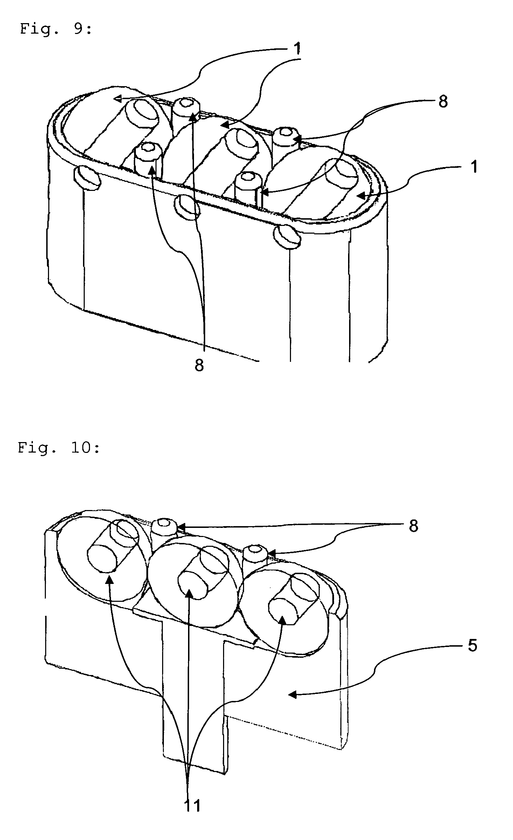

**Fig. 9 und Fig. 10** zeigen eine Vorzugsvariante, bei der drei Kugeln in einer in Reihe angeordneten sind und über eine Achse gehalten werden, wobei Fig. 9 eine Draufsicht und Fig. 10 einen Schnitt der Kugeln zeigen.

### Vorzugsvariante 5

Ø 5mm
Anzahl 3

Die drei Kugeln 1 werden jeweils über eine Achse 11 gehalten und sind in Reihe angeordnet. Die Achsen 11 verhindern nach der Montage ein Herausfallen der Kugeln 1. Die Aufheizung des Endes des Stiftes bzw. Wärmeabgabe erfolgt über entweder eine Wärmeplatte unter den Kugeln und/oder über Stempel 8, welche sich zwischen Kugel 1 und 3 befinden.

Vorteile: Das Ende kann unter fliesendes Wasser gehalten werden; Reibung der Kugeln 1 untereinander ist nicht vorhanden, Temperatureinleitung geht über eine separate Fläche (Energiezufuhr geringer) oder durch Aufheizung der Kugeln 1 von unten (Wärmeleitung geringer); Durch den Reihenaufbau kann eine min. Breite von ca. 7 mm erreicht werden.

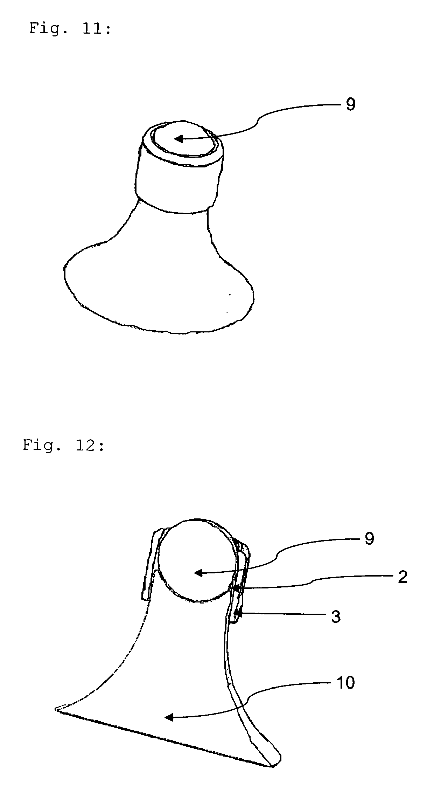

**Fig. 11 und Fig. 12** zeigen eine Vorzugsvariante, bei der eine in der Kugelpfanne 2 gelagerten Kugel (hier Bergkristall 9), beweglich ist, wobei Fig. 11 eine Draufsicht und Fig. 12 einen Schnitt der Kugeln zeigen.

### Untersuchung über die Möglichkeiten, eine Edelsteinkugel rollend in einem Kopf zu platzieren.

### (Kaltseite)

Da die Massage meisten im Gesicht bzw. bei den Augenpartien erfolgt, ist eine kleine Bauweise erwünscht. Wir haben uns für einen Kugeldurchmesser von 0 5mm entschieden da 0 von 3mm oder kleiner Schwierigkeiten im Handling bei der Monatage oder bei der Reinigung hervorrufen würden.

Die Lösung welche im ersten Abschnitt unter Lösung 3 und 5 dargestellt wurden eignen sich auch für die Kaltseite als Aufnahme für den Bergkristall 9.

### Vorzugsvariante 6 (wie Vorzugsvariante 3)

Ø 5mm

Die Kugeln laufen in einer Kugelpfanne 2. Eine Kappe 3 mit einem Loche verhindert das Herausfallen der Kugeln 1. Die Kühlung des Endes des Stiftes bzw. Kälteabgabe erfolgt über eine Kälteplatte 10 unter der Kugel 1.

Vorteile: Die Kugel 1 kann sich in alle Richtungen drehen; Kappe 3 kann zur Reinigung abgenommen werden; Das Ende kann unter fliesendes Wasser gehalten werden; Temperatureinleitung geht über die Kugeln 1 von unten.

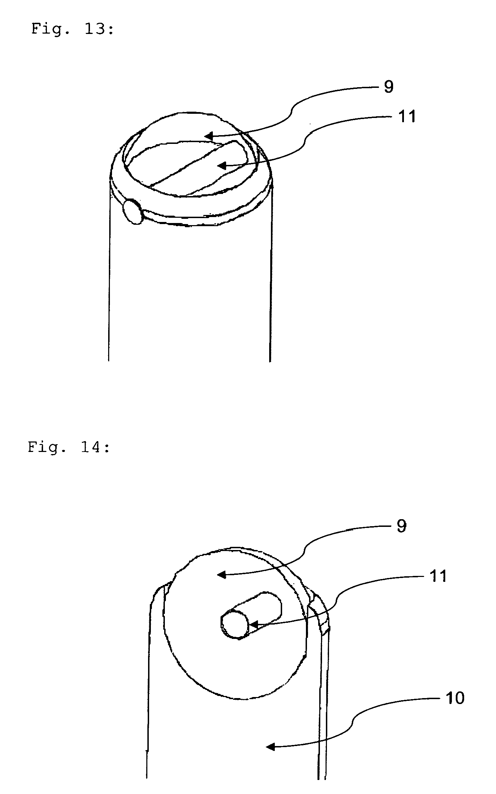

**Fig. 13 und Fig. 14** zeigen eine Vorzugsvariante, bei der Kugel 1 (hier Bergkristall 9) über eine Achse 11 gehalten wird, wobei Fig. 13 eine Draufsicht und Fig. 14 einen Schnitt der Kugeln zeigen.

### Vorzugsvariante 7 (wie Vorzugsvariante 5)

0 5mm
Anzahl 1

Die Kugel wird über eine Achse 11. Die Achse 11 verhindert nach der Montage ein Herausfallen der Kugeln. Die Kühlung des Endes des Stiftes bzw. Kälteabgabe erfolgt über eine Kälteplatte 10 unter der Kugel.

Vorteile: Das Ende kann unter fliesendes Wasser gehalten werden; Temperatureinleitung geht über eine separate Fläche.

### Informationen zu bevorzugten Temperatureinleitungen.

Da die meisten Mineralien als Isolator anzusehen sind, ist eine erhöhte Energiezufuhr nötig, um Edelsteine auf eine bestimmte Temperatur zu bringen. Eine Verbesserung der Situation wäre eine direkte Einleitung der Wärme oder Kälteenergie auf die Haut über Metallpads, welche z.B. mit Kupferkern ausgelegt sind und zur besseren Hautverträglichkeit vergoldet sind. Eine weitere Verbesserung würde man durch eine Isolierkappe erreichen, welche bis zum Erreichen der benötigten Temperatur auf dem Ende des Stiftes steckt und zur Anwendung abgenommen wird.

### Wärmeleitung am Stiftende bzw. Edelsteinkugeln.

### Vorzugsvariante 8

Für die Wärmeeinleitung am Stiftende wird ein Heizpad benötigt, welches mit einer Steuerelektronik verbunden ist (z.B. Siehe Bild eines Insektenstiftes ca. 50 C° kurzzeitig). Da die Haut eine Oberflächentemperatur von ca. 32 C° hat muss das Heizpad nur noch ca. 13 Kelvin produzieren.

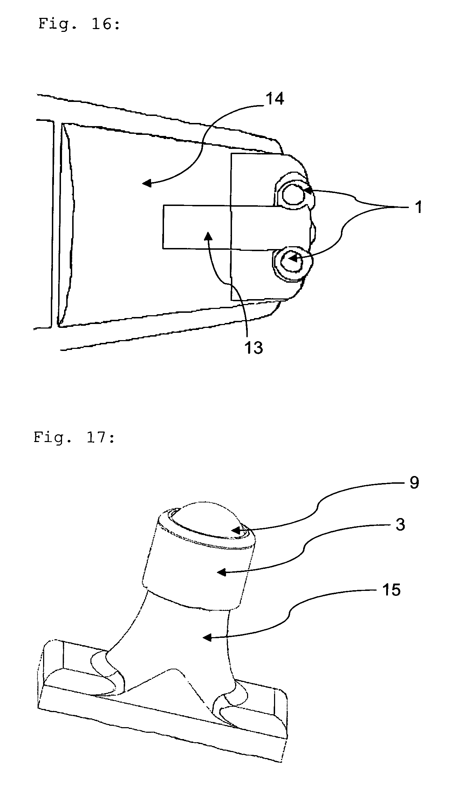

**Fig. 16** zeigt eine schematische Darstellung einer Sofort-Wärme-Kompresse.

### Vorzugsvariante 9

Im vorliegenden Anwendungs Fall würde eine Wärmekammer 14 unterhalb der Edelsteinkugeln 1 sein, welche über eine Verschraubung zu öffnen ist. Das Wärmepad würde vom Anwender aktiviert und in die Wärmekammer 14 abgelegt und geschlossen.

Das Wärmepad wärmt den Stempel unterhalb der Bergkristallkugel auf und liefert so lange Wärme bis das Wärmepad aufgebraucht ist bzw. der Anwender das Pad entfernt hat.

Vorteil: Leichte Anwendung; Kleiner Kühlaufbau; Bei der Anwendung autark; Portabel.

Beim Anwendungsfall muss keine Wärmeenergie bereitgestellt werden

**Fig. 17** zeigt eine schematische Darstellung einer Kälteeinleitung am Stiftende bzw. Edelsteinkugeln.

### Kälteeinleitung am Stiftende bzw. Edelsteinkugeln

Da die Anwendungen im hauptsächlich im Gesicht stattfindet und das Gerät von dem Benutzer selber geführt werden soll ist Lösung 10 zurückgestellt worden.

### Vorzugsvariante 10

Kleiner Kühlkompressor wie er in einem Kühlschrank Verwendung findet.

### Vorteil: Gängige Technik

### Vorzugsvariante 11

Kältekompresse (Gel) welche im Eisfach gelagert wird bzw. Stift wird im Eisfach auf die gewünschte Temperatur gekühlt.

Vorteil: Leichte Anwendung; Kleiner Kühlaufbau; Bei der Anwendung autark; Beim Anwendungsfall muss keine Kühlenergie bereitgestellt werden.

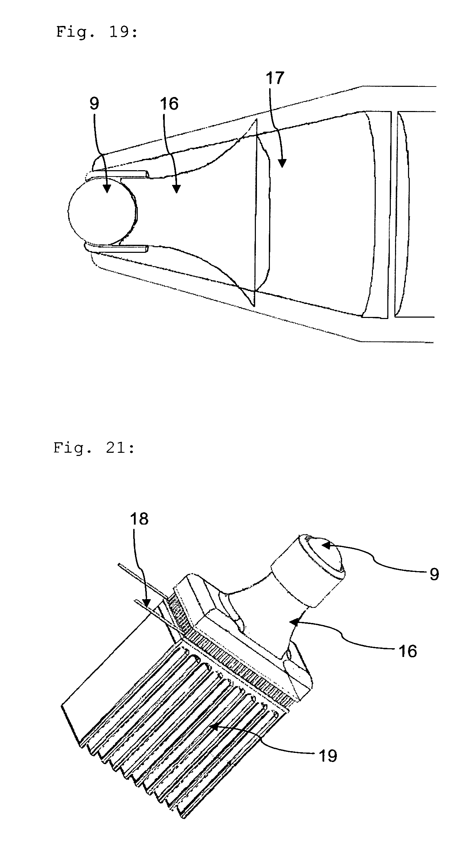

**Fig. 19** zeigt eine Sofort-Kälte-Kompresse auf Ammoniumnitrat-Basis.

### Vorzugsvariante 12

### Sofort-Kälte-Kompresse auf Amoniumnitrat-Basis

Die Abkühlung beim Lösen von Ammoniumnitrat in Wasser findet beispielsweise in Sofort-Kühlkompressen eine Anwendung. Solche Kühlkompressen kommen insbesondere bei Sportverletzungen zum Einsatz. In einer Kunststoffhülle befindet sich granuliertes Ammoniumnitrat und ein mit Wasser gefülltes Päckchen. Zur Anwendung wird die Kompresse zusammengedrückt, wobei die innere Hülle reißt und das Wasser ausläuft. Die äußere Hülle muss dabei natürlich verschlossen bleiben. Sobald das Ammoniumsalz mit Wasser in Kontakt tritt, löst es sich und es kommt zu einer starken Abkühlung. Die Kühlkompresse kann nun auf die Verletzung gelegt werden.

Im vorliegenden Anwendungsfall würde eine Kältekammer 17 unterhalb des Bergkristalls 9 sein, welche über eine Verschraubung zu öffnen ist. Das Kältepad würde vom Anwender aktiviert und in die Kältekammer 17 abgelegt und geschlossen. Das Kältepad kühlt den Stempel 16 unterhalb der Bergkristallkugel 9 ab und liefert so lange eine Kühle bis das Kältepad aufgebraucht ist bzw. der Anwender das Pad entfernt hat.

Im vorliegenden Anwendungsfall würde eine Kältekammer 17 unterhalb des Bergkristalls 9 sein, welche über eine Verschraubung zu öffnen ist. Das Kältepad würde vom Anwender aktiviert und in die Kältekammer 17 abgelegt und geschlossen. Das Kältepad kühlt den Stempel 16 unterhalb der Bergkristallkugel 9 ab und liefert so lange eine Kühle bis das Kältepad aufgebraucht ist bzw. der Anwender das Pad entfernt hat.

### Vorzugsvariante 13

Sofort-Kälte Patrone gefüllt mit Zeolith (www.zeotech.de).

Das Prinzip: Die natürlichen Rohstoffe Wasser und Zeolith werden in einem geschlossenen Adsorptionszyklus genutzt. Zum Antrieb wird lediglich Wärme benötigt. Das Verfahren ist umweltverträglich, betriebssicher und bereits in verschiedenen Anwendungsbereichen erprobt.

Im vorliegenden Anwendungsfall würde eine Kältekammer unterhalb des Bergkristalls sein, welche über eine Verschraubung zu öffnen ist. Die Zeolithkappsel würde vom Anwender aktiviert und in die Kältekammer eingelegt und geschlossen. Die Zeolithkappsel kühlt den Stempel unterhalb der Bergkristallkugel ab und liefert so lange eine Kühle bis das System aufgebraucht ist bzw. der Anwender die Kapsel entfernt hat.

Vorteil: Leichte Anwendung; Bei der Anwendung autark; Portabel; Beim Anwendungsfall muss keine Kühlenergie bereitgestellt werden.

**Fig. 21** zeigt ein Ausführungsbeispiel, bei dem ein Peltier-Element zur Kühlung benutzt wird.

### Vorzugsvariante 14

### Peltier Element

Durch das Anlegen einer Spannung an ein spezielles Halbleitermodul wird eine Wärmpumpe aktiviert welche die Wärme von einer Seite zur anderen transportiert.

Kühlt man die warme Seite z. B. mittels eines aufgesetzten Kühlkörpers mit Ventilator so wird die kühlende Seite noch kälter. Die Temperaturdifferenz zwischen den beiden Seiten kann, je nach Element und Strom, bei einstufigen Elementen bis ca. 69 Kelvin betragen. In diesem Anwendungsfall würde auf der Kaltseite des Peltierelement 18 ein isolierter Stempel geklebt, welcher die Kugelpfanne für den Bergkristall 9bildet.

Das Peltier Element 18 wird mit Gleichstrom betrieben. Als Konstruktionsbeispiel dient ein Element mit 17.1 Watt und 3,9 Ampere 7,6 Volt und einer Grundfläche von **30 x 15 x 3,9 mm.**

Auf der Warmseite muss ein Hochleistungskühlelement befestigt werden sowie eventuell zusätzlich ein Lüfter.

Vorteile: Als strombetriebenes Kühlelement derzeit kleinstmögliche Bauweise; Keine bewegten Teile; Warmseite könnte auch für die Heizphase an der Warmseite verwendet werden; Gleichstrom; Delta t von 70 Kelvin möglich; Temperaturen unter 0 °C können bei guter Wäremabfuhr erreicht werden.

Warmseite wird bei Kühlung ca. 60 bis 70C° warm.

### Vorzugsvariante 15

### Kälteerzeugung durch separates Spray

Das Stiftende mit dem Bergkristall wird mit einem Spray lokal vereist und danach zur Anwendung benutzt.

Zur Unterstützung kann ein Kältespeicher hinter dem Bergkristall positioniert werden, indem die Kälte die durch das Spray eingeleitet werden und gespeichert werden kann.

Da die Anwendung in der Nähe von Augen erfolgt, sollte ein Spray ohne Alkohol (Äther) verwendet werden.

Vorteil:Leichte Anwendung; Bei der Anwendung autark; Portabel

### Wärmeeinleitung bzw. Edelsteinführung

Für die Wärmeeinleitung sollte ein Stempel in der Mitte oder die gesammte Kappe verwendet werden, da die Energie welche unterhalb der Kugeln über Heizpads eingeleitet werde müsste, um einiges höher sein muss.

Der Mückenstift wie in Lösung 8 zeigt einen Anwendungsfall, welcher mit zwei Mignon AA Batterien als Energiequelle auskommt.

In unserem Fall bei einer minutenlangen Anwendung sollten mindestens drei Batterien der Größe AA eingesetzt werden.

Die Wärmeeinleitung sollte über eine Elektronik gesteuert werden da verschiede Phasen realisiert werden müssen.
z.B. Aufheizphase bis ca. 45°C erreicht sind
Warmhaltephase Temperatur Δ von ca. 13 Kelvin (32°C bis 45°C)

Eine weiter noch genauer zu untersuchende Möglichkeit wäre es die "Abfallwärme" des Peltier-Element für die Heizseite zu benutzen.

Ein noch zu lösendes Problem wäre die Überschusswärme, welche beim Kühlen entsteht (ca. 60°C). Es muss verhindert werden, dass diese Energie an die Warmseite gelangt (Gefahr für Benutzer).

### Kälteeinleitung bzw. Bergkristall Kühlung

Für den vorliegenden Anwendungsfall ist die Lösung 14 nach unserer Beurteilung eine machbare Lösung wodurch aber eine Baugröße durch die zu verwendeten Bauteile vorgeben ist.

Das Peltier-Element hat zwar relativ kleine Abmaße (z.B. **30 x 15 x 3,9 mm**) aber die benötigten Zusatzteile nehmen den größten Teil ein.

Kühlleiter ca. **30 x 15 x 20 mm**

Kühlerabmaße ca. **30 x 15 x 70 mm**

Der Kühler kann variabel gestaltet werden. Lüftungsschlitze sind aber nötig.

Batterie ca. Ø **36 x 105 mm bei 6 AA Zellen aber mind**. **5000mAh und ca. 5V**

Der Batteriepack kann durch ein geeignetes Netzteil ersetzt werden welches ca. die Größe eines Handynetzteiles hat.

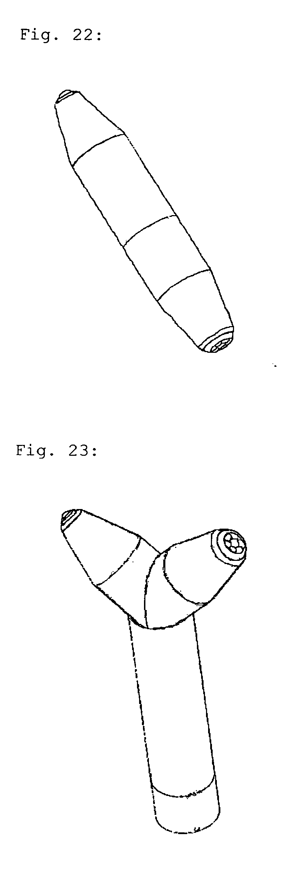

**Fig. 22 bis Fig. 24** zeigen drei konstruktive Ansichten.

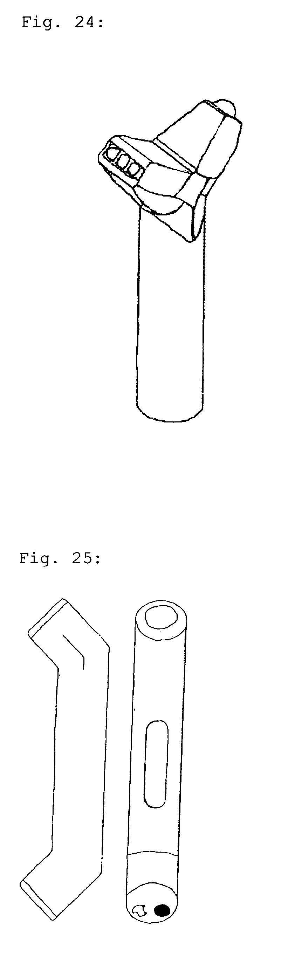

**Fig. 25** zeigt zwei Seitenansichten einer bevorzugten Ausführungsform der erfindungsgemäßen Vorrichtung in Form eines Handgerätes.

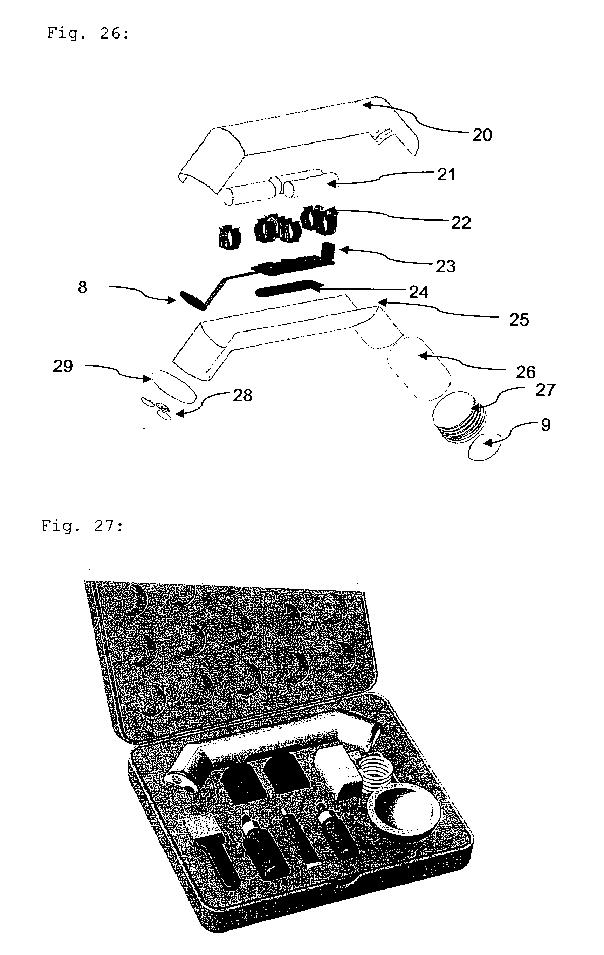

**Fig. 26** zeigt eine schematische Darstellung des technischen Konzeptes der Vorrichtung nach Anspruch 1.

**Fig. 27** zeigt einen bevorzugten Kit.

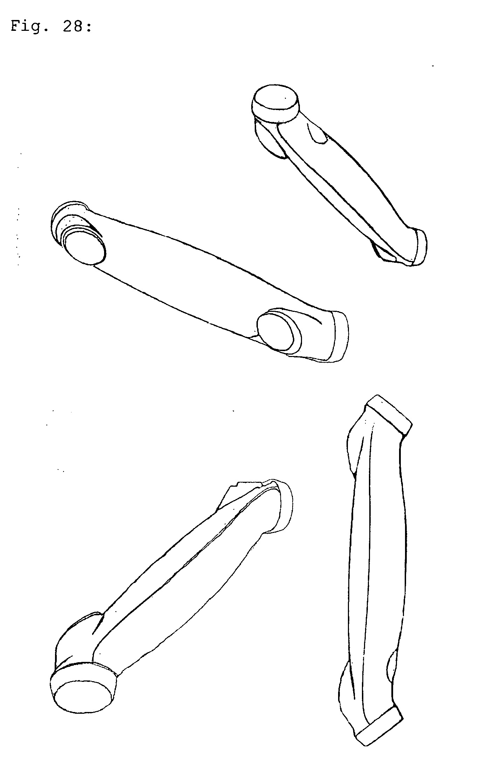

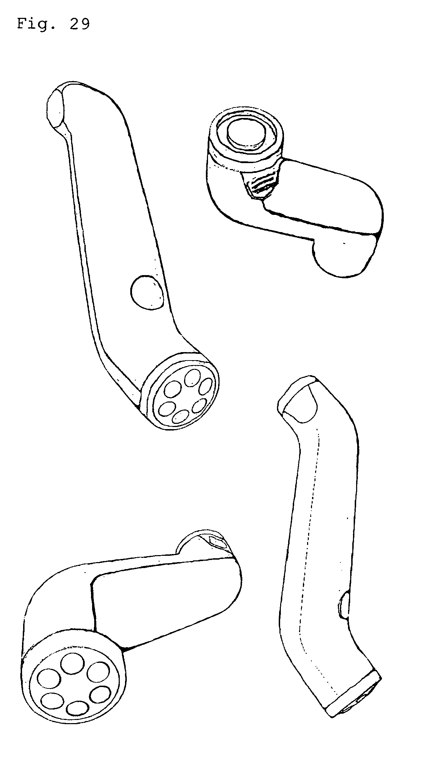

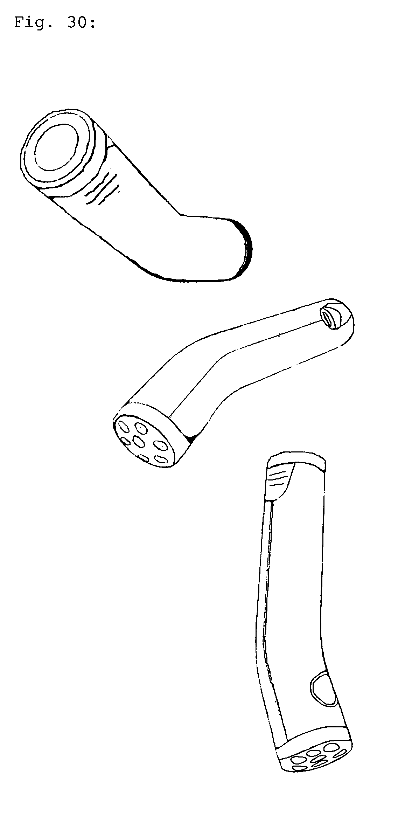

**Fig. 28 - 30** zeigen Varianten von bevorzugten Ausführungsformen der erfindungsgemäßen Vorrichtung.

### Bezugszeichenliste

- 1: Kugeln/Edelsteine
- 2: Kugelpfanne
- 3: Kappe
- 4: Heizsockel
- 5: Sockel
- 6: Heizstift
- 7: Heizpads
- 8: Heizelement
- 9: Bergkristall
- 10: Kälteplatte
- 11: Achse
- 12: Kühlsockel
- 13: Wärmeleiter
- 14: Wärmekammer
- 15: Sockel als Kühlleiter
- 16: Kälteleiter
- 17: Kältekammer
- 18: Peltier-Element
- 19: Kühler
- 20: Gehäuseschale oben
- 21: Batterieakkus
- 22: Batteriekontakte
- 23: Platine mit Ladebuchse
- 24: Silikonschalter
- 25: Gehäuseschalen unten
- 26: Dose mit Federelement
- 27: Alukappe (kalt) zum Schrauben
- 28: Ozeanachat, Aventurin, Rhodonit
- 29: Alukappe (warm)

## Patentansprüche

1. Vorrichtung zum flächenmäßig, zeitlich und mengenmäßig begrenzten Eintrag von Wärme und/oder Kälte zur kosmetischen Behandlung von Hautarealen, wobei die Vorrichtung aus einem im Wesentlichen zylindrischen Grundkörper mit zwei gegenüberliegenden Enden besteht und das eine Ende eine Kappe aus Leichtmetall umfassend Halbedelsteine oder Edelsteine aufweist und das andere Ende eine Kappe aus Leichtmetall mit einem Bergkristall umfasst und das edelsteinaufweisende Ende für eine Wärmebehandlung und das bergkristallaufweisende Ende für eine Kältebehandlung eingesetzt wird.

2. Vorrichtung nach Anspruch 1
**dadurch gekennzeichnet, dass**
die Halbedelsteine ausgewählt sind aus der Gruppe umfassend Aventurin, Rosenquarz und/oder Amethyst.

3. Vorrichtung nach einem der vorhergehenden Ansprüche,
**dadurch gekennzeichnet, dass**
die Edel- und/oder Halbedelsteine und/oder der Bergkristall drehbar gelagert sind.

4. Vorrichtung nach einem der vorhergehenden Ansprüche,
**dadurch gekennzeichnet, dass**
der begrenzte Eintrag von Wärme über eine Zeitdauer von maximal 8 Minuten erfolgt.

5. Vorrichtung nach einem der vorhergehenden Ansprüche,
**dadurch gekennzeichnet, dass**
der Bergkristall und/oder die Halbedelsteine in eine Aluminiumkappe eingebracht sind.

6. Verwendung der Vorrichtung nach einem der Ansprüche 1 bis 5 zur kosmetischen Behandlung von Hautpartien, insbesondere im Gesicht, bevorzugt von den Hautpartien, die um das Auge angeordnet sind.

7. Verwendung nach dem vorhergehenden Anspruch,
**dadurch gekennzeichnet, dass**
der Wärme- und/oder Kälteeintrag mit der Vorrichtung mit der Applikation von Serum auf der Haut kombiniert wird.

8. Verwendung nach einem der vorhergehenden Ansprüche,
**dadurch gekennzeichnet, dass**
das Serum Aminosäuren, Hyaluronsäure und Collagen umfasst.

9. Verwendung nach einem der vorhergehenden Ansprüche,
**dadurch gekennzeichnet, dass**
das Serum mithilfe des Bereichs der Vorrichtung gemäß einem der Ansprüche 1 bis 5 auf der Haut verteilt wird, die erwärmt wird.

10. Verwendung nach dem vorhergehenden Anspruch,
**dadurch gekennzeichnet, dass**
nach Abschluss der warmen Verteilung des Serums der behandelte Hautbereich mit der Vorrichtung gemäß einem der Ansprüche 1 bis 5 in Kontakt gebracht wird, der kühlt, bevorzugt die Seite, die den Bergkristall umfasst.

11. Kit umfassend die erfindungsgemäße Vorrichtung gemäß der Ansprüche 1 bis 5 und eine Emulsion oder Lösung umfassend Aminosäuren, Collagen und Hyaluronsäure.
